# EUROPEAN PATENT SPECIFICATION

(11) **EP 0 458 877 B1**
(45) Date of publication and mention of the grant of the patent: **17.05.1995**
(21) Application number: 90903643.6
(22) Date of filing: 01.02.1990
(51) Int. Cl.: A01N 1/02, A61L 27/00

(54) **CALCIFICATION MITIGATION OF BIOPROSTHETIC IMPLANTS**
VERHINDERUNG VON VERKALKUNG IN BIOPROSTHETISCHEN IMPLANTATEN
MITIGATION DE LA CALCIFICATION D'IMPLANTS BIOPROTHETIQUES

(30) Priority: 17.02.1989 US 312542; 18.04.1989 US 339787; 30.11.1989 US 444087
(43) Date of publication of application: 04.12.1991
(73) Proprietor: BAXTER INTERNATIONAL INC., Deerfield, IL 60015 (US)
(72) Inventor: CARPENTIER, Alain, F-75014 Paris (FR); CARPENTIER, Sophie, F-75014 Paris (FR); NASHEF, Aws, Huntington Beach, CA 92646 (US)
(74) Representative: MacGregor, Gordon
(86) International application number: US9000639
(87) International publication number: WO9009102

(56) References cited:
- EP-A- 0 103 946
- EP-A- 0 103 947
- EP-A- 0 172 716
- US-A- 1 892 410
- US-A- 4 097 234
- US-A- 4 378 224
- US-A- 4 648 881
- US-A- 4 729 139

## Description

### Background of the Invention

This invention relates to calcification-resistant bioprosthetic implants and to a process for preparing them. More particularly, the invention concerns calcification-resistant bioprosthetic heart valves prepared from naturally derived biological materials.

Bioprosthetic heart valves, such as glutaraldehyde preserved porcine heart valves, have overcome numerous problems associated with mechanical and homograft heart valves. These bioprosthetic valves are quite stable and non-antigenic, have a remarkable durability and have physical characteristics which closely mimic the natural valves.

An alternative technique for the tanning or preservation of animal hides and tissues involves the use of metallic salts. Chrome tanning, for example, has been employed for many years in the leather industry. (See, e.g., U.S. patent 1,892,410.) Ferric, chromium and aluminum salts have been used for tanning naturally derived collagen, which may be used for preparing implantable prosthetic articles. (See U.S. patent 4,097,234.) Dardik, et al., in U.S. patent 3,974,526, describe the preparation of bioprosthetic vascular grafts from veins and arteries obtained from the umbilical cord. Among the reagents disclosed for hardening or tanning the vessels is chromium oxide.

A recurrent problem with such bioprosthetic heart valves has been their tendency toward calcification in vivo. This problem has been particularly prevalent in children, leading some to recommend against the use of bioprosthetic heart valves in children.

Various procedures for mitigating calcification of bioprosthetic heart valves have been proposed. For example, Lentz et al., in U.S. patent 4,323,358, disclose the treatment of implantable glutaraldehyde-fixed natural tissues (including porcine heart valves) with a soluble salt of a sulfated higher aliphatic alcohol to inhibit calcification after implantation. Nimni et al., in U.S. patent 4,378,224, disclose a procedure for inhibiting calcification of bioprosthetic tissues which involves cross- linking the tissue with a sulfated protein-polysaccharide, such as chondroitin sulfate. In United States patent 4,481,009, A. S. Nashef discloses a calcification mitigation procedure which comprises incorporating a biocompatible polymer into a bioprosthetic tissue prior to implantation tissue. Dewanjee et al., in U.S. patent 4,553,974, describe a multi-step procedure for treating biological tissues to inhibit calcification. This procedure involves treating the tissue with a surfactant, washing to remove the surfactant, fixing the tissue with glutaraldehyde, treating with a calcification inhibitor, such as an amino diphosphonate and treating the tissue with a reducing agent. U.S. patent 4,648,881, Carpentier et al., teaches that calcification of implanted biological tissue can be mitigated by avoiding contact of the tissues with phosphate- containing solutions, at least during the latter stages of their preparation. This patent also teaches that treatment of the tissue with a calcium-competitive divalent cation inhibits calcification of the implanted tissue.

A need continues to exist for a convenient, reliable and effective process for preparing calcification-resistant bioprosthetic implants, such as heart valves. Such a procedure should not deleteriously affect the durability, biocompatibility or physical characteristics of the bioprosthesis, and should be amenable to use in a commercial production process.

According to the present invention there is provided a process of preparing biologically-derived tissue comprising:-
(a) treating the biologically-derived tissue with:
   i) either sufficient of a tanning solution adequately to tan the tissue; or
   ii) sufficient of a solution of a soluble, substantially non-toxic ferric salt, stannic salt or a combination thereof to mitigate calcification of the tissue *in vivo*;
(b) subsequently treating the tissue with sufficient of the other of the solutions, wherein the tanning solution is not a solution of a ferric salt, stannic salt or a combination thereof; and
(c) treating the tissue with a diamine and an activating factor for initiating reaction of the diamine with the tissue, this step being performed before step (a) or step (b)
A calcification-resistant bioprosthetic implant comprises a tanned biological material, which is impregnated with a calcification-inhibiting amount of ferric or stannic ions or a mixture thereof.

The diamine reacts with free carboxylic residues of proteins and other constituents of the tissue. The additional amine groups are free to react with either the agent used in the tanning process, or the ferric and/or stannic ions.

The activating factor is utilized with the diamine solution to initiate the reaction with the carboxylic residues. One particularly preferred activating factor is a carbodiimide.

The bioprosthetic implants produced exhibit excellent resistance to calcification for long periods of implantation. The calcification mitigation process does not deleteriously affect the physical characteristics, the durability or biocompatibility of the implants.

Calcification is a particularly troublesome problem when it occurs in prosthetic heart valves. To function effectively, such heart valves must retain the high degree of flexibility and durability of the tissues from which they are made. The valves must not only be substantially non-antigenic, thus resistant to host rejection reactions, but they also must not be thrombogenic or stimulate inflammatory reactions. Such bioprosthetic valves should provide many years of trouble-free performance. If they do fail, their failure should be attended by a gradual decrease in performance, rather than by a catastrophic loss of function.

Calcification can compromise many of these desired characteristics. Calcification reduces the flexibility of the valvular materials, and can result in sudden, unpredictable failure of the valve. Mineral deposits on the valves may cause the formation of thrombi, which, if suddenly released can cause serious complications, including cerebrovascular or cardiovascular blockage. Calcification often requires premature replacement of bioprosthetic valves, thus exposing the patient to the risks of additional surgery.

It has now been discovered that by impregnating the biological materials used for making bioprosthetic heart valves with an effective amount of ferric or stannic ions or a mixture thereof, calcification can be substantially reduced.

The heart valves that may be prepared by this procedure include all of the common types of valves prepared from natural tissues, including those prepared from porcine heart valves, bovine pericardium, human dura matter and the like.

In a preferred embodiment, the process is generally employed in combination with any suitable process of sterilizing the biological tissue. The tanning process improves the durability and reduces the antigenicity of the biological tissue.

A preferred tanning procedure is to treat the tissue with 0.625% glutaraldehyde in either phosphate-buffered solutions or phosphate- free buffers. Phosphate-free buffers are preferred, and include, for example, borate, carbonate, bicarbonate, cacodylate, and other synthetic, artificial or organic buffers, such as N-2- Hydroxyethylpiperazine-N'-2-3thanesulphonic acid (HEPES), 2-(N- morpholino)propane-sulphonic acid (MOPS) and 1,4-piperazinebis (Ethanesulphonic acid) (PIPES).

Any of a variety of sterilizing procedures can be employed for sterilizing the bioprosthetic implants. A preferred procedure includes contacting the biological tissue with a sterilization-effective amount of a solution of formaldehyde or glutaraldehyde, an alcohol and a surfactant.

The sterilization and tanning procedures may be performed before or after the calcification mitigation procedure of the invention. The aldehydes used in the tanning process covalently cross-link with protein amine groups. Cross-linking of these groups, e.g., with glutaraldehyde, is important to reduce the antigenicity of the bioprosthesis. Ferric or stannic ions are believed to form stable, covalently bound complexes or ligands with amine groups, when in the presence of an aldehyde or other substance which will form Schiff bases with the amine groups.

Thus, the reaction of the ferric and/or stannic ions with the amine groups directly competes with the tanning process. Various methods have been devised to measure the extent to which the amine groups have become cross-linked. One method of determining the extent of the cross-linking of the amine groups is measuring of the temperature at which the biological tissue experiences shrinkage (T_{S}). Natural tissue typically has a T_{S} of about 65^{o}C. As the cross-linking increases the T_{S} increases. Glutaraldehyde fixed tissue has a T_{S} of about 84oC to 85oC. This increase in hydrothermal stability of the tissue is believed to be important in terms of increased biological stability. Hence, it is desirable to insure that the impregnation with ferric and/or stannic ions does not significantly interfere with this T_{S}.

A high concentration of ferric or stannic salt may adversely affect the biological material. Such adverse effects range from a stiffening of the tissue to an actual increase in the tendency toward calcification. On the other hand, if the concentration of the ferric or stannic salt is too low, the treatment will not achieve the desired efficacy. The concentration of the ferric or stannic salt in solution may also affect the uniformity and depth of the penetration of the metallic ions throughout the biological material. In general, a uniform, deep penetration of the ferric or stannic ions in the biological material is preferred.

If the biological tissue is subjected first to the tanning solution, then care must be taken to ensure that the biological tissue can be impregnated with a calcification mitigation amount of the ferric or stannic ions. Contrarily, if the biological tissue is first subjected to the treatment with the ferric and/or stannic ion solution, care must be taken to prevent the impregnation of such a large concentration of the ferric and/or stannic ions to prevent either adequate tanning, or to cause the biological material to become stiff.

As stated, there are many factors which influence the degree of impregnation. Firstly, the concentration of the ferric or stannic ions in the impregnation solution will influence the quantity of ions available for impregnation. The extent of impregnation is influenced by the amount of contact time between the impregnation solution and the biological material, the impregnation solution pH, and the sequence of performing the impregnation process and the tanning process. These factors are controlled to obtain the desired impregnation level of ferric or stannic ion in the tissue.

The precise level of ferric and/or stannic ion impregnation for a specific biological tissue is dependent upon the desired end use. As stated, the ferric and stannic ion impregnation competes with the cross-linking action of the aldehydes during the tanning process. This will directly affect the T_{S}, which is dependent upon the degree of cross-linking obtained during the tanning process. Tissue impregnation with the ferric and/or stannic ions may also effect other characteristics of the tissue, such as flexibility of the tissue. The importance of any one characteristic is dependent upon the desired end usage for the tissue.

Accordingly, the effective amount of ferric and/or stannic ion impregnation is dependent upon the desired end usage.

Biological tissue materials used to form tissue heart valves are usually required to possess a certain degree of flexibility. As the level of ferric and/or stannic ion impregnation increases, the tissue loses flexibility. However, a sufficient amount of ferric and/or stannic ions must be incorporated to provide the necessary degree of calcification mitigation. As stated, the impregnation with ferric and/or stannic ions is highly dependent upon the concentration in the impregnating solution and the conditions under which the biological tissue material is subjected to the impregnation process.

As stated, one indicator used to measure the usefulness of biological material subjected to the tanning process is the measurement of the temperature of shrinkage (T_{S}). T_{S} is measured by holding the tissue within two clamps. One of the clamps is mounted in a manner to allow the clamp to slide in direction generally parallel to the direction in which the tissue is being held. This clamp is coupled to a device capable to indicating movement of the clamp. The tissue is immersed in a bath. The temperature of the bath is slowly raised until observing movement of the clamp.

The process of the invention is performed until the tissue is sufficiently impregnated with a calcification mitigating amount of ferric and/or stannic ions to achieve the desired calcification mitigation, while remaining sufficiently pliable for the intended end use. Once the desired degree of impregnation and pliability is obtained for a desired end use a measurement of the T_{S} provides an adequate reference point for tissue stabilization.

For example, it has been determined that biological tissue tanned using glutaraldehyde should have a T_{S} of from about 85^{o}C to possess the desired stability. Accordingly, the degree to which the tissue is impregnated with ferric and/or stannic ion should be maximized while maintaining this desired T_{S}.

The process of the invention is generally performed by contacting the biological material with a solution of a water-soluble ferric or stannic salt. This contact typically requires immersing the biological materials in the treatment solution. Alternatively, the solution may be sprayed, roll coated or applied to the biological materials using any suitable procedure. In a preferred embodiment, the biological material is immersed in the ferric or stannic salt solution for a period of time ranging from about 24 hours to about 200 hours.

The solvent or vehicle for the ferric or stannic salt may be an organic solvent system or an aqueous solvent. Examples of organic solvents which may be used include lower C₁-C₈ aliphatic alcohols, glycols, triols, aldehydes and the like.

Preferred solvent systems are aqueous solutions of water-miscible alcohols, glycols, triols and aldehydes. These aqueous solvent systems not only serve as a vehicle for the ferric or stannic salts, but also serve as protein denaturants and bacteriocidal agents. Examples of such solvent systems are aqueous solutions of ethanol, formaldehyde, glutaraldehyde, glycerol or mixtures thereof. These aqueous solvent systems have the added advantage that they have long been used in the treatment of implantable biological tissues, and their safety and effectiveness are well- established. The aqueous solvent systems advantageously contain up to about 30% ethanol, up to about 5% formaldehyde, up to about 0.625% glutaraldehyde, or up to about 30% glycerol, or mixtures thereof. These solvent systems can also advantageously contain conventional buffering agents.

The ferric or stannic ions are in the form of a soluble, substantially non-toxic salt. Examples of such salts include the nitrate, sulfate, borate, carbonate, halide, citrate and acetate salts. Preferred salts are ferric nitrate, stannic nitrate, ferric sulfate, stannic sulfate, ferric chloride, stannic chloride, ferric citrate, stannic citrate, ferric acetate and stannic acetate. Ferric salts are particularly preferred.

The ferric or stannic salt is employed in the solution in an impregnation effective concentration. Such concentration is an amount sufficient to impregnate the biological material with a calcification- mitigating amount of the ferric or stannic ions upon treatment with the solution. Generally, the concentration of the ferric or stannic salts in the solution which are applied to the tissue, will range from about 0.01% by weight to about 2.5% by weight, preferably from about 0.05% by weight to about 1.5% by weight. The ferric or stannic concentration in the tissue prior to implantation ranges from about 0.001% to about 3.0%, preferably from about 0.005% to about 1.0% by weight.

The pH of the ferric or stannic salt solution has been found to affect the rate at which the ferric and/or stannic ions are absorbed by the biological tissue. The lower the pH, the faster the absorption. The pH of the solution generally ranges from about 2 to about 7, with the pH preferably ranging from about 4.5 to about 6 being preferred.

While the invention is particularly advantageous for preparing bioprosthetic heart valves, the biological materials and processes of this invention may be applied to the preparation of other types of implants. Examples of such implants are materials for bladder reconstruction, repair of blood vessels, and orthopedic implants, to name but a few.

The invention is further illustrated by the examples below, which are not intended to be limiting.

The discovery that led to the present invention was made by accident. Porcine heart valve leaflets were treated with glycerol prior to implantation into experimental animals. The leaflets were removed after several weeks, and were observed to have significantly lower levels of calcification than expected from prior experience. In investigating the cause of this result, it was noted that the glycerol used for treating the heart valve leaflets was taken from a metal container. This container of glycerol was quite old, and the glycerol was later found to contain relatively large amounts of iron and tin. Analysis of the glycerol for metals revealed that it contained 10 parts per million ("ppm") calcium, 90 ppm iron and 30 ppm tin. Follow- up experiments suggested that the lower degree of calcification was attributable to the contaminated glycerol. A series of experiments was conducted to establish the effect of the "aged glycerol" on calcification mitigation and development of alternate solution that would achieve this effect.

### EXPERIMENT.

The following experiment is not according to the present invention, but is included to show standard tanning and calcification mitigation procedures as follows:
Freshly extracted porcine aortic heart valve leaflets were thoroughly rinsed with isotonic saline solution. The leaflets were then subjected to tanning and calcification mitigation procedures, with the calcification mitigation procedure, being performed first, although the procedure can be reversed.

The calcification mitigation procedure involved immersing the leaflets in one of two types of glycerol solutions. The first type of solution is the aged glycerol solution discussed above, while the second type of solution is commercial grade glycerol solution consisting of 1/3 ethanol (90%), 1/3 aqueous formaldehyde (prepared by mixing 4 parts 37% formaldehyde and 6 parts H₂O) and 1/3 glycerol. The latter glycerol solution was either used alone or spiked with a ferric or stannic compound. The ferric or stannic compounds used were either salts or oxides.

The tanning procedure involved immersing the leaflets in a solution consisted of 0.625 percent, on a weight/volume basis, glutaraldehyde having a pH of 7.4 ± 0.1 containing 0.02 molar N-2-Hydroxyethylpiperazine- N¹-2-Ethane- sulfonic acid (HEPES), 0.26 weight/volume percent magnesium chloride and a sufficient amount of sodium chloride to prepare an isotonic solution.

Both the tanning and calcification procedures were performed by immersing the leaflets in the respective solutions for a one week period.

After the tanning and calcification procedures were completed the leaflets were surgically implanted subcutaneously at various locations into the backs of growing rats or growing rabbits.

The calcification was measured qualitatively, which involved visual inspection and recording of the extent of calcification (results not shown).

The method of the invention provides an effective means for mitigating the calcification of tissue implants. However, one difficulty in practicing the method of the invention relates to the two reactions which compete for the free protein amine groups. That is, the calcification mitigation procedure involving the reaction between the amine groups and ferric and/or stannic ions and the tanning procedure involving the reaction between the amine groups and an aldehyde, e.g. glutaraldehyde. The balancing of these two reactions directly affects the final properties of the tissue.

For example, if the calcification mitigation procedure is not regulated, the concentration of the ferric and/or stannic ions binding with the amine groups will restrict the available amine sites for reaction with the aldehyde which will stiffen the final tissue product. If the tanning procedure is not regulated, not enough amine groups will be available for binding with the ferric and/or stannic ions, thus negating any calcification mitigation effect.

In accordance with the invention a diamine is introduced into the tissue in the presence of an activating factor. Diamines bind to the free carboxylic residues on proteins and mucopolysaccharides present in the tissue, and in particular the tissue collagen. A more detailed explanation of the coupling of diamines to carboxylic residues and useful activating factors is disclosed in Journal of Polymer Science: Polymer Chemistry Edition, by Lloyd and Burns, Vol. 17, pp. 3459-3489 (1979), which is generally incorporated herein by reference and specifically with respect to the useful types of activating factors.

The treatment of fixed tissue with a diamine is also taught in U.S. Patent Nos. 4,729,139, issued on March 8, 1988, and 4,481,009, issued on November 6, 1984, both of which are issued to Aws Nashef. The incorporation of diamines in tissue, as disclosed by the procedures discussed in these two patents is to assist in the covalent bonding of monomers, which are subsequently polymerized, to the carboxylic residues of proteins and mucopolysaccharides of tanned tissue. The general purpose of the procedures taught by these two patents is the inhibition of calcification in implantable tissue through the impregnation of the tissue with a polymer.

The diamine may be applied as the first step of the process. That is, a diamine is applied before either the tanning procedure or the calcification mitigation procedure. This provides additional amine groups for linkage with the aldehyde and the ferric and/or stannic ions. Alternatively, the diamine may be applied in between the first and second steps of the invention. That is, the diamine may be applied after the tanning procedure and before the calcification mitigation procedure, or vice versa depending upon the sequence of the two primary steps.

The preferred process of the invention involves tanning the tissue and then subsequently performing the calcification mitigation procedure. In this preferred embodiment the application of the diamine is performed between the tanning and the calcification mitigation steps.

The diamine is typically applied to the tissue in an aqueous solution, with the diamine being water soluble. Preferred diamines are those having the formula R-(NH2)2, wherein R is a cyclic or noncyclic alkyl, aryl, alkylene, arylalkyl, arylalkylene radical having from one to ten carbons. The selected diamine should readily diffuse through the tissue protein network and preferably be water soluble. A particularly preferred diamine is ethylenediamine.

The activating factor is typically added directly to the aqueous solution containing the diamine. Accordingly, the useful activating factors are also water soluble. The preferred activating factor is one or more types of carbodiimides, with a particularly preferred activating factor being 1-ethyl-3(3-dimethylaminopropyl)- carbodiimide-HCL.

Typically, the tissue is soaked in the aqueous solution containing the diamines and the activating factors for about five to about thirty minutes, with the pH of the solution adjusted to about 4.75.

The concentration of the diamine should be selected to ensure the availability of at least one-hundred fifty moles of diamine to every one mole of collagen present in the tissue. This molar ratio is theorized to ensure the effective binding of diamines to available carboxylic residues. This theory is based upon the assumption that one mole of collagen exists for every 100,000 grams dry, weight of tissue, and that one hundred fifty carboxylic residues are present for every one mole of collagen.

The amount of activating factor applied with the diamine is also dependent upon this theorized concentration of free carboxylic acid residues in the collagen. The amount of activating factor used is generally at least greater than about five times the theorized number of available carboxylic acid residues in the collagen.

In the examples below, after the tanning and calcification procedures were completed the leaflets were surgically implanted subcutaneously at various locations into the backs of growing rats. These leaflets were left implanted for those periods of time indicated for each example. Three leaflets were implanted in each rat, with two rats being sacrificed for each time interval. After the rats were sacrificed the leaflets were removed and observed for calcification.

The calcification was measured qualitatively in the Examples. The qualitative procedure used involved visual inspection and recording of the extent of calcification using a calcification scale of: no observable calcification (0), some calcification (+), average amount of the calcification (++) or severe calcification (+++). A letter F indicates minute degree of calcification observed under microscope. A letter E indicates that calcification was in the cells only, and represents only minor calcification. Generally, the samples being observed at each time interval possessed different degrees of calcification. As seen in the following charts, two different indications are given for each sample. This represents different degrees of calcification between the six different leaflets. However, except for certain examples, marked accordingly, no attempt was made to record the precise number of leaflets having each type of calcification. First, an X-ray of each sample was observed to provide a general indication of calcification. If a greater degree of differentiation was needed then a leaflet section was stained and examined for calcification. The stained section was prepared by embedding the leaflets in a paraffin block, sectioning the blocks and staining the sections with Von Kossa stain.

### EXAMPLES 1-4

The following examples demonstrate the invention. The results of Examples 1-4 are shown in the following tables 1-4, with the Treatment Codes E and C designating the Experimentally treated tissue and the Control Tissue.

### EXAMPLE 1

The following example demonstrates the treatment of implantable porcine heart valve leaflets with ethylenediamine (ED). The valve tissue was previously fixed by treatment with glutaraldehyde-HEPES. The treatment with ED was performed prior to the calcification treatment with ferric nitrate in a 5% aqueous formaldehyde solution.

Test leaflets were treated for one week with glutaraldehyde/HEPES buffer (as described above). Next the leaflets were soaked in an aqueous solution containing 0.1 molar ED. This aqueous solution also contained 5 grams of 1-ethyl-3(3 dimethylaminopropyl)-carbodiimide- HCL per grams dry weight of tissue. The pH of the solution was adjusted to 4.75 while the leaflets soaked for thirty minutes. The leaflets were then rinsed in saline and soaked for one week in 5% aqueous formaldehyde containing 0.1% ferric nitrate, then for three weeks in glutaraldehyde/HEPES buffer without ferric nitrate. The leaflets were sterilized by soaking in 4% formaldehyde for twelve hours at room temperature followed by rinsing in 0.625% glutaraldehyde/HEPES buffered saline (pH 7.4). Control leaflets were tanned with the glutaraldehyde/HEPES buffer solution and were sterilized with FETH. The calcium and iron contents of the test leaflets prior to implantation were 0.005% and 0.93% respectively. The calcium and iron contents of the control leaflets prior to implantation were 0.019% and 0.024% respectfully. The leaflets were implanted in rabbits, then removed and analyzed after a specified period of time in accordance with the procedures discussed above. The period of time for each test was three, six, nine and twelve weeks as stated in the following Table 1. The calcium and iron contents and standard deviations for the removed leaflets are shown in Table 1. This experiment demonstrates that the sequential treatment with ED and with ferric nitrate in aqueous formaldehyde results in substantial calcification mitigation.

**TABLE 1**

| Rabbit # | Treatment Code | Implant time (weeks) | | | | | | | |
|---|---|---|---|---|---|---|---|---|---|
| | | 3 | | 6 | | 9 | | 12 | |
| | | Ca | Fe | Ca | Fe | Ca | Fe | Ca | Fe |
| 1 | E | 0.121 | 0.895 | -- | -- | -- | -- | STUDY TERMINATED | |
| | C | -- | -- | -- | -- | -- | -- | | |
| 2 | E | 0.112 | 0.763 | 1.293 | 0.382 | 0.135 | 0.539 | | |
| | C | 0.113 | 0.004 | -- | -- | 0.194 | 0.055 | | |
| 3 | E | 0.073 | 0.593 | 0.171 | 0.558 | 0.116 | 0.568 | | |
| | C | 0.214 | 0.749 | 0.418 | 0.112 | 1.771 | 0.041 | | |
| 4 | E | 1.745 | 0.606 | 0.209 | 0.541 | 1.100 | 0.557 | | |
| | C | -- | -- | -- | -- | 0.109 | 0.017 | | |
| 5 | E | 0.120 | 0.651 | 0.140 | 0.587 | 0.196 | 0.719 | | |
| | C | 0.124 | 0 | 0.218 | 0.036 | 0.441 | 0.021 | | |
| 6 | E | 0.124 | 0.570 | 2.486 | 0.302 | 0.201 | 0.604 | | |
| | C | 0.048 | 0 | 0.230 | 0.049 | 0.211 | 0.017 | | |
| | E | 0.382± 0.668 | 0.680± 0.126 | 0.860± 1.030 | 0.474± 0.125 | 0.350± 0.421 | 0.597± 0.072 | | |
| | | n = 6 | n = 6 | n = 5 | n = 5 | n = 5 | n = 5 | | |
| Mean ± S.D. | C | 0.125± 0.068 | 0.188± 0.374 | 0.289± 0.112 | 0.066± 0.041 | 0.545± 0.696 | 0.030± 0.017 | | |
| | | n = 4 | n = 4 | n = 3 | n = 3 | n = 5 | n = 5 | | |

### EXAMPLE 2

The following example demonstrates the treatment of previously fixed implantable porcine heart valve leaflets first with ethylenediamine (ED) and then with ferric nitrate in an aqueous formaldehyde/ethanol solution (calcification mitigation).

Test leaflets were treated for one week with glutaraldehyde/HEPES buffer. The leaflets were then incubated in 0.1 M ED solution, adjusted to a pH of 4.75 for thirty minutes followed by the addition of five grams of 1-ethyl- 3(3 dimethylaminopropyl)-carbodiimide- HCL per gram (dry weight) of tissue). The reaction proceeded at a pH of 4.75 for thirty minutes. The leaflets were subsequently rinsed in saline(0.9%). The leaflets were then treated for one week in an aqueous solution containing 5% formaldehyde, 30% ethanol and 0.1% ferric nitrate, then for three weeks in a glutaraldehyde/HEPES buffer solution without ferric nitrate. The leaflets were sterilized with 4% formaldehyde for twelve hours at room temperature followed by rinsing in 0.625% HEPES/glutaraldehyde solution maintained at a pH of 7.4. Control leaflets were tanned with the glutaraldehyde/HEPES buffer solution and were sterilized with FETH. The calcium and iron contents of the test leaflets prior to implantation were 0.006% and 1.983% respectively. The calcium and iron contents of the control leaflets prior to implantation were 0.019% and 0.024% respectfully. The leaflets were implanted in rabbits, then removed and analyzed after a specified period of time. The period of time for each test was three, six, nine and twelve weeks as stated in the following Table 2. The calcium and iron contents and standard deviations for the removed leaflets are shown in Table 2. This experiment demonstrates that the sequential treatment with ED and with ferric nitrate in aqueous formaldehyde/ethanol results in substantial calcification mitigation.

**TABLE 2**

| Rabbit # | Treatment Code | Implant time (weeks) | | | | | | | |
|---|---|---|---|---|---|---|---|---|---|
| | | 3 | | 6 | | 9 | | 12 | |
| | | Ca | Fe | Ca | Fe | Ca | Fe | Ca | Fe |
| 1 | E | -- | -- | -- | -- | -- | -- | STUDY TERMINATED | |
| | C | 0.120 | 0.002 | -- | -- | -- | -- | | |
| 2 | E | 0.941 | 1.047 | 0.424 | 1.691 | 0.327 | 1.850 | | |
| | C | 0.139 | 0 | -- | -- | 0.167 | 0.060 | | |
| 3 | E | 0.196 | 1.892 | -- | -- | -- | -- | | |
| | C | -- | -- | -- | -- | -- | -- | | |
| 4 | E | 0.195 | 1.938 | 0.263 | 1.223 | 2.447 | 1.620 | | |
| | C | 0.123 | 0 | 0.183 | 0.024 | 4.873 | 0.018 | | |
| 5 | E | 0.167 | 1.965 | -- | -- | 0.222 | 1.968 | | |
| | C | 1.243 | 0 | -- | -- | 2.838 | 0.033 | | |
| 6 | E | 0.188 | 1.951 | -- | -- | 0.365 | 2.109 | | |
| | C | 0.154 | 0 | 0.236 | 0.046 | 03.829 | 0.045 | | |
| | E | 0.337± 0.338 | 1.759± 0.399 | 0.344± 0.114 | 1.457± 0.331 | 0.840± 1.073 | 1.887± 0.207 | | |
| | | n = 5 | n = 5 | n = 2 | n = 2 | n = 4 | n = 4 | | |
| Mean ± S.D. | C | 0.356± 0.496 | 0.0004± 0.001 | 0.210± 0.037 | 0.035± 0.016 | 2.927± 2.019 | 0.039± 0.018 | | |
| | | n = 5 | n = 5 | n = 2 | n = 2 | n = 4 | n = 4 | | |

### EXAMPLE 3

The following example demonstrates the treatment of implantable porcine heart valve leaflets first fixed with glutaraldehyde/HEPES buffer and then treated with ethylenediamine (ED) prior to the calcification treatment with ferric nitrate in an aqueous formaldehyde/glycerol solution.

Test leaflets were treated for one week with glutaraldehyde/HEPES buffer. They were then soaked in an aqueous solution (pH 7.45) of 0.1 molar ED and 5 grams of 1- ethyl-3(3 dimethylaminopropyl)-carbodiimide- HCL/gram (dry weight) of tissue for thirty minutes, with the leaflets subsequently rinsed in saline. The leaflets were then treated for one week in an aqueous solution of 5% formaldehyde, 24% glycerol and 0.1% ferric nitrate, then for three weeks in a glutaraldehyde/HEPES buffered solution without ferric nitrate. The leaflets were sterilized with 4% formaldehyde in HBS for twelve hours at room temperature as described for Examples 1 and 2. Control leaflets were tanned with the glutaraldehyde/HEPES buffer solution and were sterilized with FETH. The calcium and iron contents of the test leaflets prior to implantation were 0.004% and 0.307% respectively. The calcium and iron contents of the control leaflets prior to implantation were 0.019% and 0.024% respectfully. The leaflets were implanted in rabbits, then removed and analyzed after a specified period of time in accordance with the procedures discussed above. The period of time for each test was three, six, nine and twelve weeks as stated in the following Table 3. The calcium and iron contents and standard deviations for the removed leaflets are shown in Table 3. This experiment demonstrates that the sequential treatment with ED and with ferric nitrate in aqueous formaldehyde/glycerol results in substantial calcification mitigation.

**TABLE 3**

| Rabbit # | Treatment Code | Implant time (weeks) | | | | | | | |
|---|---|---|---|---|---|---|---|---|---|
| | | 3 | | 6 | | 9 | | 12 | |
| | | Ca | Fe | Ca | Fe | Ca | Fe | Ca | Fe |
| 1 | E | -- | -- | 0.109 | 0.163 | -- | -- | -- | -- |
| | C | 0.079 | 0.088 | -- | -- | -- | -- | -- | -- |
| 2 | E | 0.110 | 0.345 | -- | -- | -- | -- | -- | -- |
| | C | 0.098 | 0 | -- | -- | -- | -- | -- | -- |
| 3 | E | 0.080 | 0.249 | 0.162 | 0.167 | 0.173 | 0.161 | 2.474 | 0.109 |
| | C | 0.264 | 0 | 1.775 | 0.004 | 1.320 | 0.038 | 1.149 | 0.053 |
| 4 | E | 0.084 | 0.140 | 1.035 | 0.146 | 0.238 | 0.197 | 0.346 | 0.150 |
| | C | 0.200 | 0.084 | 0.896 | 0.024 | 0.228 | 0.037 | -- | -- |
| 5 | E | 0.064 | 0.116 | -- | -- | -- | -- | 0.165 | 0.172 |
| | C | -- | -- | 0.162 | 0.014 | 0.240 | 0.052 | 0.234 | 0.021 |
| 6 | E | 0.083 | 0.186 | 0.162 | 0.147 | 0.805 | 0.061 | 3.411 | 0.117 |
| | C | 0.083 | 0 | 0.271 | 0.031 | 0.199 | 0.043 | 0.856 | 0.055 |
| | E | 0.084± 0.016 | 0.207± 0.092 | 0.367± 0.446 | 0.156± 0.011 | 0.405± 0.348 | 0.140± 0.070 | 1.599± 1.600 | 0.137± 0.029 |
| | | n = 5 | n = 5 | n = 4 | n = 4 | n = 3 | n = 3 | n = 4 | n = 5 |
| Mean ± S.D. | C | 0.145± 0.083 | 0.034± 0.047 | 0.776± 0.740 | 0.018± 0.012 | 0.497± 0.549 | 0.042± 0.007 | 0.746± 0.467 | 0.043± 0.019 |
| | | n = 5 | n = 5 | n = 4 | n = 4 | n = 4 | n = 4 | n = 3 | n = 3 |

### EXAMPLE 4

The following example demonstrates the treatment of implantable porcine heart valve leaflets first fixed with glutaraldehyde/HEPES buffer and then treated with ethylenediamine (ED) prior to the calcification treatment with ferric nitrate in an aqueous ethanol/formaldehyde/glycerol.

Test leaflets were treated for one week with glutaraldehyde/HEPES buffer. They were then soaked in an aqueous solution (pH 7.45) of 0.1 molar ED and 5 grams of 1- ethyl-3(3 dimethylaminopropyl)-carbodiimide- HCL/gram (dry weight) of tissue for thirty minutes, with the leaflets subsequently rinsed in saline. The leaflets were then treated for one week in an aqueous solution of ethanol/formaldehyde/reagent grade glycerol containing 0.1% ferric nitrate, then for three weeks with the glutaraldehyde/HEPES buffer without ferric nitrate. The leaflets were sterilized with 4% formaldehyde in HBS for twelve hours at room temperature as described for Examples 1 and 2. Control leaflets were tanned with the glutaraldehyde/HEPES buffer solution and were sterilized with FETH. The calcium and iron contents of the test leaflets prior to implantation were 0.007% and 1.609% respectively. The calcium and iron contents of the control leaflets prior to implantation were 0.019% and 0.024% respectfully. The leaflets were implanted in rabbits, then removed and analyzed after a specified period of time in accordance with the procedures discussed above. The period of time for each test was three, six, nine and twelve weeks as stated in the following Table 4. The calcium and iron contents and standard deviations for the removed leaflets are shown in Table 4. These experiments demonstrate that the sequential treatment with ED and with ferric nitrate in aqueous ethanol/formaldehyde/glycerol results in substantial calcification mitigation.

**TABLE 4**

| Rabbit # | Treatment Code | Implant time (weeks) | | | | | | | |
|---|---|---|---|---|---|---|---|---|---|
| | | 3 | | 6 | | 9 | | 12 | |
| | | Ca | Fe | Ca | Fe | Ca | Fe | Ca | Fe |
| 1 | E | 0.339 | 1.625 | 0.491 | 1.365 | 0.558 | 1.288 | 0.477 | 1.398 |
| | C | 0.105 | 0.006 | 0.561 | 0.025 | 0.248 | 0.062 | 6.637 | 0.037 |
| 2 | E | -- | -- | 0.471 | 1.425 | 0.467 | 1.459 | 0.617 | 1.249 |
| | C | 0.195 | 0.004 | -- | -- | 3.429 | 0.073 | 0.731 | 0.024 |
| 3 | E | 0.295 | 1.642 | 0.607 | 1.499 | -- | -- | 0.729 | 1.307 |
| | C | 0.049 | 0 | 0.195 | 0.042 | 1.402 | 0.045 | -- | -- |
| 4 | E | 0.267 | 1.757 | 0.572 | 1.539 | 0.748 | 1.280 | 0.571 | 1.700 |
| | C | 0.133 | 0.001 | 2.495 | 0.027 | 6.291 | 0.069 | 0.731 | 0.024 |
| 5 | E | 0.177 | 1.331 | 0.456 | 1.416 | -- | -- | 0.729 | 1.307 |
| | C | 0.089 | 0 | 0.199 | 0.013 | 1.871 | 0.015 | -- | -- |
| 6 | E | 0.269 | 1.403 | 0.665 | 1.595 | -- | -- | -- | -- |
| | C | 0.111 | 0.017 | 0.263 | 0.015 | -- | -- | -- | -- |
| | E | 0.269± 0.059 | 1.552+ 0.178 | 0.544± 0.084 | 1.473± 0.086 | 0.591± 0.143 | 1.342± 0.101 | 0.625± 0.108 | 1.392± 0.180 |
| | | n = 5 | n = 5 | n = 6 | n = 6 | n = 3 | n = 3 | n = 5 | n = 5 |
| Mean ± S.D. | C | 0.144± 0.049 | 0.005± 0.006 | 0.743± 0.991 | 0.024± 0.012 | 2.648± 2.334 | 0.053± 0.024 | 2.700± 3.410 | 0.028± 0.008 |
| | | n = 6 | n = 6 | n = 5 | n = 5 | n = 5 | n = 5 | n = 3 | n = 3 |

## Claims

1. A process of preparing biologically-derived tissue comprising:-
(a) treating the biologically-derived tissue with:
i) either sufficient of a tanning solution adequately to tan the tissue; or
ii) sufficient of a solution of a soluble, substantially non-toxic ferric salt, stannic salt or a combination thereof to mitigate calcification of the tissue *in vivo*;
(b) subsequently treating the tissue with sufficient of the other of the solutions, wherein the tanning solution is not a solution of a ferric salt, stannic salt or a combination thereof; and
(c) treating the tissue with a diamine and an activating factor for initiating reaction of the diamine with the tissue, this step being performed before step (a) or step (b)

2. The process of claim 1 wherein the tissue is treated with said diamine and said activating factor as a single aqueous solution.

3. The process of claim 1 or 2 wherein said activating factor is a carbodiimide.

4. The process of claim 3 wherein said carbodiimide is 1-ethyl-3(3-dimethylaminopropyl)-carboidimide-HCL.

5. The process of any preceding claim wherein the salt is a nitrate, sulfate, borate, carbonate, halide, citrate or acetate of ferric or stannic.

6. The process of claim 5 wherein the salt is ferric nitrate, stannic nitrate, ferric sulfate, stannic sulfate, ferric chloride, stannic chloride, ferric citrate, stannic citrate, ferric acetate or stannic acetate.

7. The process of claim 5 or 6 wherein the salt is dissolved in an aqueous solution of water-miscible alcohol, glycol, triol, or aldehyde.

8. The process of claim 7 wherein the salt is dissolved in an aqueous solution of ethanol, formaldehyde, glutaraldehyde, or glycerol.

9. The process of claim 8 wherein the solution contains up to about 30% ethanol, up to about 5% formaldehyde, up to about 1% glutaraldehyde, or up to about 30% glycerol, or mixtures thereof.

10. The process of any preceding claim wherein the pH of said ferric and/or stannic salt solution ranges from about 2 to about 7.

11. The process of claim 10 wherein the pH of said ferric or stannic salt solution ranges from about 2 to about 3.

12. The process of any preceding claim wherein the said ferric or stannic salt solution contains from about 0.01% to about 2.5% by weight of the ferric or stannic salt.

13. The process of claim 12 wherein the said ferric or stannic salt solution contains from about 0.05% to about 15% of the ferric or stannic salt.

14. The process of any preceding claim wherein said diamine is selected from those having the formula:
R-(NH₂)₂
Wherein: R is a C₁-C₁₀ cyclic or noncylic alkyl, aryl, alkylene, arylalkyl, arylalkylene radical.

15. The process of claim 14 wherein R is a straight chain aliphatic group.

16. The process of any preceding claim wherein said diamine is ethylenediamine.

17. The process of any preceding claim wherein said biological tissue is in the form of a porcine heart valve.

18. The process of any preceding claim wherein said tissue is treated with said diamine at a rate of one-hundred fifty moles diamine to every one mole of collagen of said tissue.

19. The process of any preceding claim wherein said tissue is treated with said activating factor at a rate of five times the amount of carboxylic residues of collagen of said tissue.

## Patentansprüche

1. Verfahren zum Präparieren von biologisch erhaltenem Gewebe, wobei das Verfahren folgende Schritte aufweist:
(a) Behandeln des biologisch erhaltenen Gewebes mit:
(i) entweder genügend von einer Gerblösung, um das Gewebe adäquat zu gerben; oder
(ii) genügend von einer Lösung aus einem löslichen, im wesentlichen nichttoxischen Eisen(III)-salz, Zinn(IV)-salz oder einer Kombination davon, um die Verkalkung des Gewebes *in vivo* zu mildern;
(b) anschließendes Behandeln des Gewebes mit genügend der jeweils anderen der Lösungen, wobei die Gerblösung keine Losung aus einem Eisen(III)-salz, einem Zinn(IV)-salz oder einer Kombination davon ist; und
(c) Behandeln des Gewebes mit einem Diamin und einem Aktivierungsfaktor zur Einleitung der Reaktion des Diamins mit dem Gewebe, wobei dieser Schritt vor Schritt (a) oder Schritt (b) durchgeführt wird.

2. Verfahren nach Anspruch 1, wobei das Gewebe mit dem Diamin und dem Aktivierungsfaktor als einer einzigen wäßrigen Lösung behandelt wird.

3. Verfahren nach Anspruch 1 oder 2, wobei der Aktivierungsfaktor ein Carbodiimid ist.

4. Verfahren nach Anspruch 3, wobei das Carbodiimid 1-Ethyl-3(3-dimethylaminopropyl)carbodiimid-HCL ist.

5. Verfahren nach einem der vorhergehenden Ansprüche, wobei das Salz ein Nitrat, Sulfat, Borat, Carbonat, Halogenid, Citrat oder Acetat von Eisen(III) oder Zinn(IV) ist.

6. Verfahren nach Anspruch 5, wobei das Salz Eisen(III)-nitrat, Zinn(IV)-nitrat, Eisen(III)-sulfat, Zinn(IV)-sulfat, Eisen(III)-chlorid, Zinn(IV)-chlorid, Eisen(III)-citrat, Zinn(IV)-citrat, Eisen(III)-acetat oder Zinn(IV)-acetat ist.

7. Verfahren nach Anspruch 5 oder 6, wobei das Salz in einer wäßrigen Lösung von mit Wasser mischbarem Alkohol, Glykol, Triol oder Aldehyd gelöst wird.

8. Verfahren nach Anspruch 7, wobei das Salz in einer wäßrigen Lösung von Ethanol, Formaldehyd, Glutaraldehyd oder Glycerin gelöst wird.

9. Verfahren nach Anspruch 8, wobei die Lösung bis zu ca. 30 % Ethanol, bis zu ca. 5 % Formaldehyd, bis zu ca. 1 % Glutaraldehyd oder bis zu ca. 30 % Glycerin oder Gemische davon enthält.

10. Verfahren nach einem der vorhergehenden Ansprüche, wobei der pH der Eisen(III)- und/oder Zinn(IV)-salzlösung im Bereich von ca. 2 bis ca. 7 liegt.

11. Verfahren nach Anspruch 10, wobei der pH der Eisen(III)-oder Zinn(IV)-salzlösung im Bereich von ca. 2 bis ca. 3 liegt.

12. Verfahren nach einem der vorhergehenden Ansprüche, wobei die Eisen(III)- oder Zinn(IV)-salzlösung ca. 0,01 Gew.-% bis ca. 2,5 Gew.-% des Eisen(III)- oder Zinn(IV)-salzes enthält.

13. Verfahren nach Anspruch 12, wobei die Eisen(III)- oder Zinn(IV)-salzlösung ca. 0,05 % bis ca. 15 % des Eisen(III)- oder Zinn(IV)-salzes enthält.

14. Verfahren nach einem der vorhergehenden Ansprüche, wobei das Diamin unter denjenigen mit der folgenden Formel:
R-(NH₂)₂
ausgewählt ist, wobei R eine C₁-C₁₀ aufweisende cyclische oder nichtcyclische Alkyl-, Aryl-, Alkylen-, Arylalkyl-, Arylalkylengruppe ist.

15. Verfahren nach Anspruch 14, wobei R eine geradkettige aliphatische Gruppe ist.

16. Verfahren nach einem der vorhergehenden Ansprüche, wobei das Diamin Ethylendiamin ist.

17. Verfahren nach einem der vorhergehenden Ansprüche, wobei das biologische Gewebe in Form einer Schweineherzklappe ist.

18. Verfahren nach einem der vorhergehenden Ansprüche, wobei das Gewebe mit dem Diamin in einer Menge von 150 mol Diamin pro 1 mol Collagen des Gewebes behandelt wird.

19. Verfahren nach einem der vorhergehenden Ansprüche, wobei das Gewebe mit dem Aktivierungsfaktor in einer Menge entsprechend der fünffachen Menge von Carbonsäuregruppen von Collagen des Gewebes behandelt wird.

## Revendications

1. Procédé de préparation d'un tissu d'origine biologique dans lequel
a) on traite le tissu d'origine biologique avec :
i) soit une quantité suffisante d'une solution de tannage adéquate pour tanner le tissu ; soit
ii) une quantité suffisante d'une solution d'un sel ferrique soluble sensiblement non toxique, d'un sel stannique ou l'une de leur combinaison pour atténuer la calcification du tissu in vivo ;
b) ensuite on traite le tissu avec une quantité suffisante de l'autre solution, la solution de tannage n'étant pas une solution d'un sel ferrique, d'un sel stannique ou une de leur combinaison, et
c) on traite les tissus avec une diamine et un facteur activant pour amorcer la réaction de la diamine avec le tissu, cette étape étant réalisée avant l'étape (a) ou l'étape (b)

2. Procédé de la revendication 1 dans lequel on traite le tissu avec ladite diamine et ledit facteur activant sous la forme d'une seule solution aqueuse.

3. Procédé de la revendication 1 ou 2 dans lequel ledit facteur activant est un carbodiimide.

4. Procédé de la revendication 3 dans lequel ledit carbodiimide est le chlorhydrate de 1-éthyl-3(3-diméthylaminopropyl)-carbodiimide.

5. Procédé de l'une quelconque des revendications précédentes dans lequel le sel est un nitrate, sulfate, borate, carbonate, halogénure, citrate ou acétate ferrique ou stannique.

6. Procédé de la revendication 5 dans lequel le sel est le nitrate ferrique, le nitrate stannique, le sulfate ferrique, le sulfate stannique, le chlorure ferrique, le chlorure stannique, le citrate ferrique, le citrate stannique, l'acétate ferrique ou l'acétate stannique.

7. Procédé de la revendication 5 ou 6 dans lequel on dissout le sel dans une solution aqueuse d'un alcool glycol, triol, ou aldéhyde miscible à l'eau.

8. Procédé de la revendication 7 dans lequel on dissout le sel dans une solution aqueuse d'éthanol, de formaldéhyde, de glutaraldéhyde ou de glycérol.

9. Procédé de la revendication 8 dans lequel la solution contient jusqu'à environ 30 % d'éthanol, jusqu'à environ 5 % de formaldéhyde, jusqu'à environ 1 % de glutaraldéhyde, ou jusqu'à environ 30 % de glycérol, ou leurs mélanges.

10. Procédé de l'une quelconque des revendications précédentes, dans lequel le pH de ladite solution de sel ferrique et stannique est compris entre environ 2 et environ 7.

11. Procédé de la revendication 10 dans lequel le pH de ladite solution de sel ferrique ou sel stannique est compris entre environ 2 et environ 3.

12. Procédé de l'une quelconque des revendications précédentes dans lesquelles ladite solution de sel ferrique ou stannique contient d'environ 0,01 % à environ 2,5 % en poids du sel ferrique ou stannique.

13. Procédé de la revendication 12 dans lequel ladite solution de sel ferrique ou stannique contient d'environ 0,05 % à environ 15 % du sel ferrique ou stannique.

14. Procédé de l'une quelconque des revendications précédentes dans lequel ladite diamine est choisie parmi celles qui répondent à la formule :
R-(NH₂)₂
dans laquelle R est un radical alkyle, aryle, alkylène, arylalkyle, arylalkylène cyclique ou non cyclique en C₁ à C₁₀.

15. Procédé de la revendication 14 dans lequel R est un groupe aliphatique à chaîne droite.

16. Procédé de l'une quelconque des revendications précédentes dans lesquelles ladite diamine est l'éthylène diamine .

17. Procédé de l'une quelconque des revendications précédentes dans lequel ledit tissu biologique est sous la forme d'une valvule cardiaque de porc.

18. Procédé de l'une quelconque des revendications précédentes dans lequel on traite ledit tissu avec ladite diamine à raison de 150 moles de diamine pour chaque mole de collagène dudit tissu.

19. Procédé de l'une quelconque des revendications précédentes dans lequel on traite ledit tissu avec ledit facteur activant à raison de 5 fois la quantité de résidus carboxyliques de collagène dudit tissu.
